Europäisches Patentamt

(19) European Patent Office

Office européen des brevets

(11) Publication number: 0 219 027
A2

## (12) EUROPEAN PATENT APPLICATION

(21) Application number: 86113808.9

(22) Date of filing: 06.10.86

(51) Int. Cl.⁴: **C 12 N 15/00**
C 12 N 1/20, C 12 P 13/06
C 12 P 13/08
//(C12N1/20, C12R1:13, 1:15)

(30) Priority: 04.10.85 JP 221424/85

(43) Date of publication of application:
22.04.87 Bulletin 87/17

(84) Designated Contracting States:
DE FR GB

(71) Applicant: KYOWA HAKKO KOGYO CO., LTD.
6-1, Ohte-Machi Itchome
Chiyoda-ku Tokyo(JP)

(72) Inventor: Katsumata, Ryoichi
Popuraoka Coopu 6-401, 2-12-3, Naruse
Machida-shi Tokyo(JP)

(72) Inventor: Mizukami, Toru
1-6-16, Asahi-machi
Machida-shi Tokyo(JP)

(72) Inventor: Oka, Tetsuo
2360-17, Nara-machi Midori-ku
Yokohama-shi Kanagawa-ken(JP)

(74) Representative: Vossius & Partner
Siebertstrasse 4 P.O. Box 86 07 67
D-8000 München 86(DE)

(54) Process for producing amino acids.

(57) Productivity of L-lysine, L-threonine and L-isoleucine in coryneform bacteria is enhanced by transforming a microorganism of the genus *Corynebacterium* or *Brevibacterium* with a plasmid constructed by inserting a DNA fragment containing the gene responsible for the synthesis of aspartate semialdehyde dehydrogenase or aspartate aminotransferase in a microorganism of *Corynebacterium* or *Brevibacterium* into a vector. The transformant produces enhanced quantities of the desired amino acid upon culturing in a medium.

EP 0 219 027 A2

Croydon Printing Company Ltd

Our Ref: U 909 EP
Case: 434-EPC
Kyowa Hakko Kogyo Co., Ltd.                October 6, 1986
Tokyo, Japan

# PROCESS FOR PRODUCING AMINO ACIDS

Aspartate semialdehyde dehydrogenase (EC 1.2.1.11: referred to as ASD hereinafter) is an enzyme catalyzing the biosynthesis from β-aspartyl phosphate to aspartic β-semialdehyde (referred to as ASA hereinafter), and plays an important role in the production of amino acids such as lysine, threonine and isoleucine of which ASA is a precursor. Aspartate aminotransferase (EC 2.6.1.1: referred to as AAT hereinafter) is an enzyme catalyzing the reaction wherein an amino group is transferred to oxalacetic acid, and plays an important role in the supply of aspartic acid. This enzyme is also important in the production of amino acids such as lysine, threonine and isoleucine whose precursor is aspartic acid. The present invention relates to a process for producing L-lysine, L-threonine or L-isoleucine by introducing a recombinant DNA comprising a gene coding for ASD or AAT and a vector DNA into a coryneform bacterium such as a microorganism of the genus Corynebacterium or Brevibacterium and culturing the bacterium in a medium. The present invention provides an effective means for producing the above-mentioned amino acids which are particularly useful in medicines, animal feed and food.

Heretofore, in the production of L-lysine by fermentation using coryneform bacteria, strains having a homoserine requiring mutation and/or a lysine analog-resistant mutation have been employed. In the production of L-threonine and L-isoleucine, strains having a threonine analog-resistant mutation and those having isoleucine analog-resistant mutation respectively are used. See, e.g. Nakayama, K. In G. Reed (ed.), Prescott and Dunn's Industrial Microbiology, 4th ed. p.748 (1982), AVI Publishing Co., Westport, Conn.

In commonly owned U.S. patent applications Serial No. 868,705, filed May 30, 1986 and Serial No. 836,989, filed March 6, 1986, processes for producing lysine, threonine or isoleucine by constructing a recombinant DNA which contains a DNA fragment coding for a gene responsible for the biosynthesis of the amino acid in coryneform bacteria are described.

With the recent increase in the demand for such amino acids as lysine, threonine and isoleucine, improved processes for the industrial production thereof are desired. To this end, improved strains have been prepared by recombinant DNA technology which produce lysine, threonine and isoleucine in high yield.

In accordance with the present invention, the amino acids L-lysine, L-threonine and L-isoleucine are produced by transforming a coryneform bacterium with a recombinant

DNA comprising a vector DNA and a DNA fragment including a gene coding for ASD or AAT originating from a microorganism belonging to the genus Corynebacterium or Brevibacterium.

The present invention thus provides an improved process for producing L-lysine, L-threonine or L-isoleucine which comprises transforming a microorganism belonging to the genus Corynebacterium or Brevibacterium with a recombinant DNA comprising a vector DNA and a DNA fragment carrying genetic information responsible for the synthesis of ASD or AAT of a microorganism belonging to the genus Corynebacterium or Brevibacterium; culturing the transformant in a nutrient medium until recoverable amounts of L-lysine, L-threonine or L-isoleucine are accumulated in the culture broth and recovering the amino acid therefrom.

Fig. 1 illustrates the cleavage map of plasmid pASD2 for the restriction enzymes BglII, BamHI, and SalI and the steps for constructing pASD2.

Fig. 2 illustrates the cleavage map of plasmid pAT1 for the restriction enzymes BglII, BamHI and SalI and the steps for constructing pAT1.

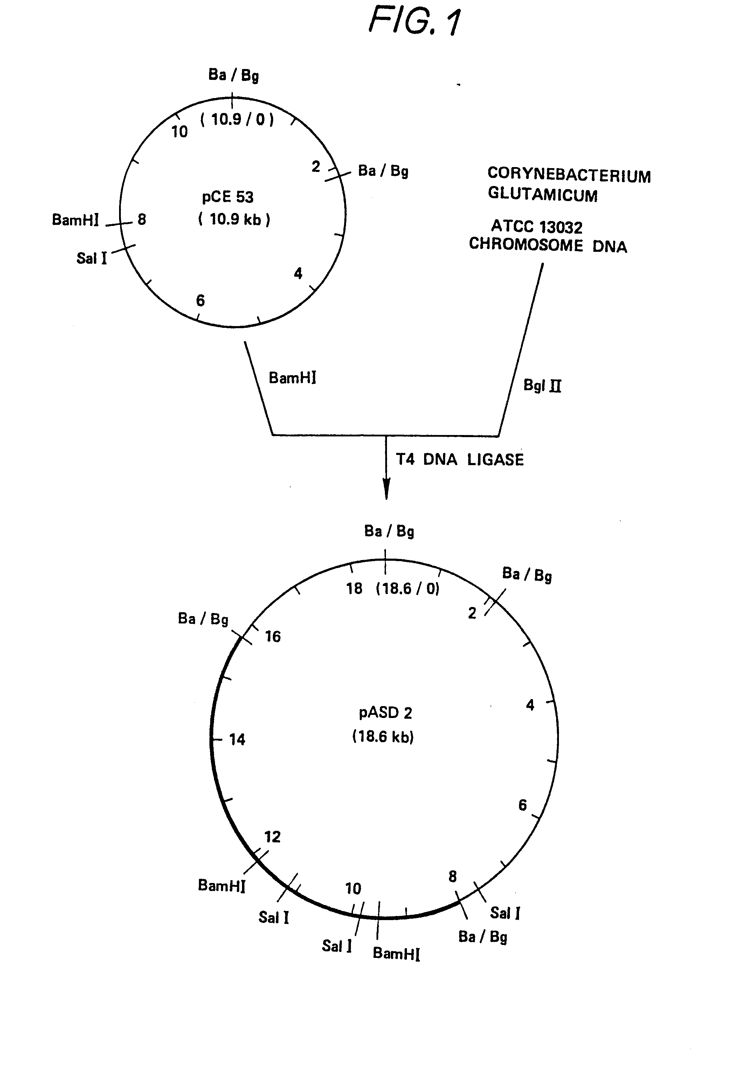

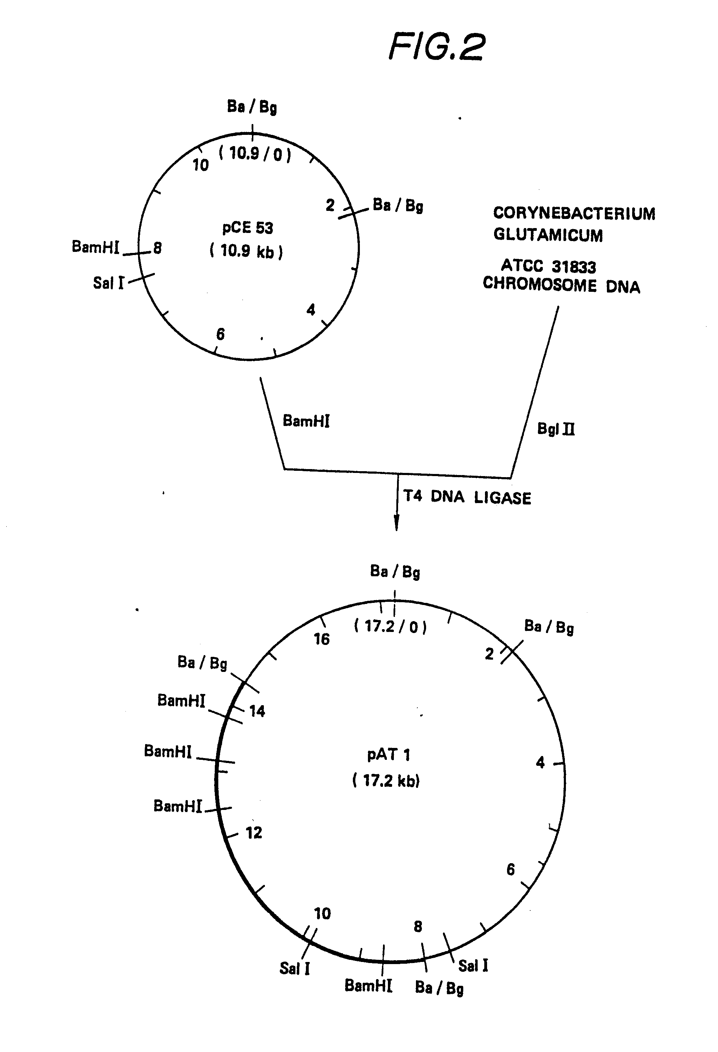

In Figs. 1 and 2, the molecular size of the plasmids is given in kilobases (kb); plasmids pASD2 and pAT1 each contain ASD and AAT genes as a DNA fragment originating from chromosome DNA indicated by the heavy line. The junction at the same cohesive ends of BamHI and BglII is shown as Ba/Bg.

In accordance with the invention, a coryneform bacterium is transformed with a recombinant DNA constructed from a vector DNA and a gene coding for ASD or AAT.

As a host microorganism, all the microorganisms known as the coryneform bacteria such as strains of the genus Corynebacterium or Brevibacterium can be used. The following strains are exemplary of those suitable for transformation:

| | | |
|---|---|---|
| Corynebacterium | glutamicum | ATCC 13032 |
| Corynebacterium | glutamicum | ATCC 31833 |
| Corynebacterium | acetoacidophilum | ATCC 13870 |
| Corynebacterium | herculis | ATCC 13868 |
| Corynebacterium | lilium | ATCC 15990 |
| Brevibacterium | divaricatum | ATCC 14020 |
| Brevibacterium | flavum | ATCC 14067 |
| Brevibacterium | immariophilum | ATCC 14068 |
| Brevibacterium | lactofermentum | ATCC 13869 |
| Brevibacterium | thiogenitalis | ATCC 19240 |
| Microbacterium | ammoniaphilum | ATCC 15354 |

Preferably, lysine-, threonine- or isoleucine-producing mutant strains derived from the above noted microorganisms are used. These mutant strains can be constructed by conventional mutation methods to confer analog-resistance, for example.

As a source of the gene coding for ASD or AAT, any microorganism can be used so long as it is a microorganism belonging to the genus Corynebacterium or Brevibacterium and has ASD or AAT activity. For exmaple, Corynebacterium glutamicum ATCC 13032 or Corynebacterium glutamicum ATCC 31833 are used.

- 5 -

0219027

Generally, the chromosome DNA used as a supply source of ASD or AAT genes can be isolated from microorganisms having ASD or AAT activity according to the procedure as disclosed in Japanese Published Unexamined Patent Application No. 126789/83 and described in the following Examples.

The vector to be used in the invention is restricted only to the extent that it is autonomously replicable in a coryneform bacterium. Particularly suited plasmids are: pCG1 described in U.S. Patent Application Serial No. 573,591 filed January 25, 1984; pCG2 described in U.S. Patent No. 4,489,160; pCG4 and pCG11 described in U.S. Patent No. 4,500,640; pCE54 and pCB101 described in Japanese Published Unexamined Patent Application No. 105999/83; pCE51 described in Japanese Published Unexamined Patent Application No. 34197/85; pCE52 and pCE53 described in Molecular and General Genetics 196, 175 (1984).

Generally, a recombinant of an ASD gene and a vector DNA can be prepared by transforming an ASD-deficient mutant derived from a strain of coryneform bacteria according to ordinary mutation procedures with the mixture of in vitro recombinants of the chromosome DNA and the vector DNA and by selecting a homoserine- and diaminopimelic acid-non-requiring transformant. Specifically, when a shuttle vector autonomously replicable in both coryneform bacteria and Escherichia coli such as pCE53 is employed, the recombinant DNA can be introduced into the coryneform bacteria after the ASD gene is cloned by transforming the ASD-deficient mutant strain of Escherichia coli with the recombinant plasmid DNA to complement the deficiency in ASD.

In like fashion a recombinant of an AAT gene and a vector DNA can be prepared by transforming an AAT deficient mutant strain derived from a strain of coryneform bacteria according to ordinary mutation procedures with a mixture of in vitro recombinants of the chromosome DNA and the vector DNA, and by selecting an aspartic acid-non-requiring transformant. Specifically, when a shuttle vector autonomously replicable in both coryneform bacteria and Escherichia coli such as pCE53 is used, the recombinant DNA can be introduced into the coryneform bacteria after the AAT gene is cloned by transforming the aminotransferase deficient mutant strain of Escherichia coli with the recombinant DNA to complement the deficiency in aminotransferase.

Transformation of the coryneform bacteria with the recombinant DNA is conveniently carried out according to a method using protoplasts developed by the present inventors as described in the following Examples and Japanese Published Unexamined Patent Application Nos. 186492/82 and 186489/82.

Production of L-lysine, L-threonine or L-isoleucine by the transformants is generally carried out according to conventional culturing processes for producing these amino acids by fermentation. That is, by culturing the transformant in an ordinary medium containing a carbon source, a nitrogen source, inorganic compounds, amino acids, vitamins, etc. under aerobic conditions while adjusting the temperature, pH, etc., the desired amino acids are accumulated in the culture broth, and may be recovered therefrom by standard procedures.

As the carbon source, carbohydrates such as glucose, glycerol, fructose, sucrose, maltose, mannose, starch, starch hydrolyzate, molasses, etc.; polyalcohols; and various organic acids such as pyruvic acid, fumaric acid, lactic acid, acetic acid, etc. can be used. Furthermore, hydrocarbons, alcohols etc. can be used depending upon the assimilability of the microorganism to be used. Particularly, cane molasses is preferably used.

As the nitrogen source, ammonia; various ammonium salts such as ammonium chloride, ammonium sulfate, ammonium carbonate, ammonium acetate, etc.; urea and other nitrogen containing materials as well as various nitrogen-containing organic materials such as peptone, NZ-amine, meat extract, yeast extract, corn steep liquor, casein hydrolyzate, fish meal or its digested product, chrysalis hydrolyzate, and the like can be used.

As inorganic materials, dipotassium hydrogen phosphate, potassium dihydrogen phosphate, magnesium sulfate, sodium chloride, ferrous sulfate, manganese sulfate, calcium carbonate, and the like are appropriate. Naturally, if vitamins, amino acids, etc. required for the growth of the microorganism are supplied by other medium components described above, it is not necessary to add these specific nutrients separately to the medium.

Culturing is generally carried out under aerobic conditions, for example, by shaking culture or by aeration-stirring culture. The preferred culturing temperature is generally 20 to 40°C; and the pH of the medium is preferably maintained around neutrality. Usually, after culturing for 1 to 5 days under these conditions recoverable quantities of L-lysine, L-threonine or L-isoleucine are accumulated in the broth. The cells are removed from the culture liquor and then the amino acid accumulated is recovered from the culture broth according to known procedures, for example, by activated carbon treatment or by ion exchange resin treatment.

By employing the present invention, L-lysine, L-threonine or L-isoleucine can be produced in higher yield using a coryneform bacterium transformant harboring a recombinant DNA containing a DNA fragment that contains the ASD or AAT gene, compared to a microorganism containing no recombinant DNA.

In the following examples of the present invention, one mode of improving L-lysine-, L-threonine- or L-isoleucine-producing ability is given, namely, the gene coding for ASD or AAT originating from Corynebacterium glutamicum is introduced into an L-lysine-, L-threonine- or L-isoleucine-producing strain of Corynebacterium glutamicum to enhance the ability to produce these amino acids. However, it is known that genes of a bacterium belonging to the genus Corynebacterium and vector plasmids likewise operate in a bacterium of the genus Brevibacterium, as the present inventors previously disclosed in Japanese Published Unexamined Patent Application No. 183799/82 and Japanese Published Unexamined Patent Application No. 210994/85. See also U.S. Patents No. 4,489,160 and No. 4,500,640. Accordingly, it is manifest that the present invention can be applied to production of these amino acids by using a bacterium of the genus Brevibacterium.

Certain specific embodiments of the present invention are illustrated by the following representative examples.

Example 1

(1) Cloning of a gene coding for ASD of Corynebacterium glutamicum

Cloning was carried out in the host-vector system of Escherichia coli K12 strain. The chromosome DNA used as a supply source of ASD gene was isolated from Corynebacterium glutamicum ATCC 13032 according to the following method disclosed in Japanese Published Unexamined Patent Application No. 126789/83. In such method, a seed culture of Corynebacterium glutamicum ATCC 13032 grown in NB medium (a medium containing 20 g of bouillon powder and 5 g of yeast extract in 1 ℓ of deionized water, and adjusted to pH 7.2) was inoculated into 400 ml of semisynthetic medium SSM [a medium containing 20 g of glucose, 10 g of $(NH_4)_2SO_4$, 3 g of urea, 1 g of yeast extract, 1 g of $KH_2PO_4$, 0.4 g of $MgCl_2 \cdot 6H_2O$, 10 mg of $FeSO_4 \cdot 7H_2O$, 0.2 mg of $MnSO_4 \cdot 4-6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6Mo_7O_{24} \cdot 4H_2O$, 30 µg of biotin and 1 mg of thiamine hydrochloride in 1 ℓ of water, and adjusted to pH 7.2] and was subjected to shaking culture at 30°C. The optical density at 660 nm (OD) was determined with a Tokyo Koden colorimeter and when the OD reached 0.2, penicillin G was added to a final concentration of 0.5 unit/ml. Culturing was continued until the OD reached 0.6.

Then, the cells were collected from the culture broth and washed with TES buffer solution [0.03M Tris-(hydroxymethyl) aminomethane (referred to as Tris hereinafter), 0.005M EDTA (disodium ethylenediaminetetraacetate), and 0.05M NaCl, and adjusted to pH 8.0 with HCl]. The washed cells were suspended in 10 ml of a lysozyme solution (25%

sucrose, 0.1M NaCl, 0.05M Tris-HCl and 0.8 mg/ml lysozyme, pH 8.0; hereinafter referred to as LS solution) and reacted at 37°C for 4 hours. The cells treated with lysozyme were collected and chromosome DNA was isolated from the cells according to the method of Saito and Miura, Biochem. Biophys. Acta, 72, 619 (1963).

As the vector plasmid, shuttle plasmid pCE53 described in Mol. Gen. Genet, 196, 175 (1984) of Escherichia coli and Corynebacterium glutamicum was used. This plasmid has multiple drug resistance (kanamycin, tetracycline, chloramphenicol, ampicillin), and is prepared by inserting a BglII-cleaved fragment of vector plasmid pCG1 described in U.S. Patent Application Serial No. 573,591 into vector plasmid pGA22 of Escherichia coli described in J. Bacteriol., 140, 400 (1979) and deposited as Escherichia coli K57 FERM BP-898 with the Fermentation Research Institute (FRI), Agency of Industrial Science and Technology on September 4, 1985 at a restriction enzyme BamHI cleavage site around the gene coding for kanamycin resistance (see Fig. 1).

The plasmid pCE53 was isolated from Escherichia coli K12 containing pCE53 according to the method of G. An, et al., described in J. Bacteriol. 140, 400 (1979). First, 8 units of restriction enzyme BamHI (a product of Takara Shuzo Co.) was added to 50 μℓ of a reaction solution for restriction enzyme (10 mM Tris-HCl, 6 mM $MgCl_2$, 100 mM NaCl and 2 mM 2-mercaptoethanol, pH 8.0) containing 4 μg of pCE53 plasmid DNA prepared above, and the mixture was subjected to reaction at 37°C for 60 minutes. Similarly, 8 units of restriction enzyme BglII (Takara Shuzo Co.) was added to 50 μℓ of the restriction enzyme reaction solution containing 4 μg of the chromosome DNA, and the mixture was reacted at 37°C for 60 minutes.  Each reaction mixture was heated at 65°C for 10 minutes to stop the reactions.  Both reaction mixtures were then mixed, and 30 µℓ of a buffer solution for T4 ligase (660 mM Tris-HCl, 66 mM $MgCl_2$ and 100 mM dithiothreitol, pH 7.6), 30 µℓ of 5 mM ATP, one unit of T4 ligase (Takara Shuzo Co.) and 140 µℓ of $H_2O$ were added thereto.  The mixture was incubated at 4°C for 24 hours, and then heated at 65°C for 10 minutes to stop the reaction.

The ligase reaction mixture was used for transformation. As the recipient, an ASD-deficient and host-specificity restriction-deficient mutant strain K58 ($F^-$ asd hsdR) derived from Escherichia coli K12 strain was used.  The K58 strain was deposited as FERM BP-899 with the FRI on September 4, 1985.  The competent cells of the strain were prepared according to the method of M. Dagert, et al., Gene 6, 23 (1979).

To 0.2 ml of a suspension containing $10^9$/ml competent cells was added 100 µℓ of the above-prepared ligase reaction mixture and incubation was carried out for 10 minutes under ice-cooling.  The reaction mixture was warmed at 37°C for 5 minutes, and 2 ml of LB medium (a medium containing 10 g of Bacto tryptone, 5 g of yeast extract, 5 g of sodium chloride and 1 g of glucose in one ℓ of water, and adjusted to pH 7.2) was added thereto. The mixture was then incubated at 30°C for 120 minutes and the cells were washed twice with physiological saline solution and separated by centrifugation.  The cells were spread on M9 plate medium (a medium containing 2 g of glucose, 1 g of $NH_4Cl$, 6 g of $Na_2HPO_4$, 3 g of $KH_2PO_4$, 0.1 g of $MgSO_4 \cdot 7H_2O$, 15 mg of $CaCl_2 \cdot 2H_2O$, 4 mg of thiamine hydrochloride and 15 g of agar in 1 ℓ of water, and adjusted to pH 7.2). Each colony grown on the M9 plate medium was spread on LB plate medium containing 100 µg/ml ampicillin or 20 µg/ml tetracycline. Colonies grown on the medium containing ampicillin and incapable of growing on the medium containing tetracycline were selected.

Plasmids were isolated from the selected transformants according to the above-mentioned method of G. An, et al. Analysis by digestion with various restriction enzymes and agarose gel electrophoresis confirmed that the plasmid pASD2 obtained from one of the transformants was a plasmid wherein a BglII DNA fragment of about 7.7 kb was inserted at the single BamHI cleavage site of pCE53 as illustrated in Fig. 1.

Thereafter, a transformant of the K58 strain after retransformation with pASD2 DNA showed homoserine- and diaminopimelic acid non-requirement establishing that the ASD gene originating from Corynebacterium glutamicum was cloned into the pASD2 plasmid.

(2)  Incorporation of pASD2 plasmid into Corynebacterium glutamicum RH6 strain

Corynebacterium glutamicum RH6 FERM BP-704 which is a lysine-producing strain was cultured with shaking in NB medium at 30°C for 16 hours. The resulting culture broth was inoculated in SSM medium containing 200 µg/ml homo-serine and cultured with shaking at 30°C. When the OD reached 0.2, penicillin G was added to a final concentration of 0.5 unit/ml. Culturing was continued and when the OD reached 0.6, the cells were collected and suspended in a solution containing 1 mg/ml lysozyme in RCGP medium comprising 5 g of glucose, 5 g of casamino acid, 2.5 g of

- 13 -

0219027

yeast extract, 1.5 g of $KH_2PO_4$, 3.5 g of $K_2HPO_4$, 0.41 g of $MgCl_2 \cdot 6H_2O$, 10 mg of $FeSO_4 \cdot 7H_2O$, 2 mg of $MnSO_4 \cdot 4-6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6Mo_7O_{24} \cdot 4H_2O$, 30 µg of biotin, 2 mg of thiamine hydrochloride, 135 g of disodium succinate, and 30 g of polyvinyl pyrolidone K15 having an average molecular weight of 10,000 (made by Tokyo Kasei Co.) in 1 ℓ of water, adjusted to pH 7.3 to make up about $10^9$ cells/ml. The suspension was transferred to an L-tube and subjected to gentle shaking culture at 30°C for 14 hours to induce protoplasts. Then, 0.5 ml of the protoplast suspension was taken into a small test tube, and centrifuged at 2,500 x g for 5 minutes. The protoplasts were suspended again in 1 ml of TSMC buffer solution (10 mM $MgCl_2$, 30 mM $CaCl_2$, 50 mM Tris-HCl and 400 mM sucrose, pH 7.5), washed by centrifugation, and then suspended in 0.1 ml of TSMC buffer solution.

Then, 100 µℓ of a 1 : 1 mixture of TSMC buffer solution at a two-fold concentration and the pASD2 plasmid DNA solution was added to the cell suspension, and 0.8 ml of TSMC buffer solution containing 20% polyethyleneglycol (PEG) 6,000 (made by Nakarai Kagaku Yakuhin Co.) was added thereto. After 3 minutes, 2 ml of RCGP medium was added, and the mixture was centrifuged at 2,500 x g for 5 minutes. The precipitated protoplasts were suspended in 1 ml of RCGP medium and incubated at 30°C for 2 hours. Then, 0.2 ml of the protoplast suspension was spread onto RCGP medium containing 1.4% agar and 300 µg/ml kanamycin, and cultured at 30°C for 10 days. A strain capable of growing on NB agar medium containing 20 µg/ml kanamycin was obtained from the resultant colonies.

A plasmid was isolated from the kanamycin-resistant transformant according to the following method which is also described in Japanese Published Unexamined Patent Application No. 134500/82. First, the transformant was cultured with shaking in NB medium at 30°C for 16 hours. The culture broth was then inoculated into 400 ml of SSM medium and cultured with shaking at 30°C. When the OD reached 0.2, penicillin G was added to a final concentration of 0.5 unit/ml and culturing was continued until the OD reached 0.6. After the cells were collected and washed with TES buffer solution, they were suspended in 10 ml of an LS solution and allowed to react at 37°C for 2 hours. Then, 2.4 ml of 5M NaCl, 0.6 ml of 0.5M EDTA (pH 8.5), and 4.4 ml of a solution consisting of 4% sodium laurylsulfate and 0.7M NaCl were added in order to the reaction solution, and the thus obtained mixture was gently mixed and placed on ice water for 15 hours. The reaction solution was then centrifuged at 69,400 x g at 4°C for 60 minutes to recover a supernatant. Then, PEG 6,000 in an amount corresponding to 10% by weight was added thereto, and the mixture was gently stirred for dissolution. The solution was placed on ice water, and after 10 hours, centrifuged at 1,500 x g for 10 minutes to remove the supernatant. Then, 5 ml of TES buffer solution was added to gently redissolve the precipitate, and 2.0 ml of 1.5 mg/ml ethidium bromide was added thereto. Cesium chloride was added to adjust the density of the solution to 1.580.

The solution was subjected to ultracentrifugation at 105,000 x g at 18°C for 48 hours. A fluorescent band at a high density level at the lower position of the centrifuge tube detected under ultraviolet irradiation was withdrawn to separate the plasmid DNA. The thus obtained fraction was treated 5 times with an equal volume of an isopropyl alcohol solution consisting of 90% by volume isopropyl alcohol and 10% by volume TES buffer solution to remove ethidium bromide by extraction, and then dialyzed against TES buffer solution to obtain a final plasmid DNA preparation. As a result of analysis by digestion with various restriction enzymes and agarose gel electrophoresis, it was determined that the plasmid had the same structure of pASD2.

(3) Production of lysine by <u>Corynebacterium glutamicum</u> RH6 containing pASD2

L-lysine production tests of the transformant RH6/pASD2 obtained by the above steps and its parent strain containing no plasmid were carried out. First, 0.5 ml of a seed culture obtained by shaking culture in NB medium at 30°C for 16 hours was inoculated in 5 ml of a production medium L1 containing 100 g of glucose, 30 g of $(NH_4)_2SO_4$, 0.5 g of $KH_2PO_4$, 0.5 g of $K_2HPO_4$, 1 g of $MgSO_4 \cdot 7H_2O$, 10 mg of $FeSO_4 \cdot 7H_2O$, 10 mg of $MnSO_4 \cdot 4-6H_2O$, 100 μg of biotin, 200 mg of homoserine and 30 g of calcium carbonate in 1 ℓ of water, adjusted to pH 7.2, and cultured with shaking at 30°C for 72 hours. After culturing, the amount of the L-lysine produced in the culture filtrate was determined by colorimetry according to the acid copper-ninhydrin method [J. Biol. Chem., <u>199</u>, 91 (1952)]. The results are shown in Table 1 and confirm that the recombinant plasmid pASD2 containing the gene coding for ASD has an effect of increasing L-lysine productivity.

<u>TABLE 1</u>

| <u>Strain</u> | <u>L-lysine (mg/ml)</u> |
|---|---|
| <u>Corynebacterium glutamicum</u> RH6 | 14.8 |
| <u>Corynebacterium glutamicum</u> RH6/pASD2 | 17.7 |

- 16 -

0219027

(4)  Production of threonine by <u>Corynebacterium</u> <u>glutamicum</u>
ATCC 21660 containing pASD2

In the same manner as step (2), the threonine-producing
strain <u>Corynebacterium</u> <u>glutamicum</u> ATCC 21660 was cultured
in SSM medium containing 200 µg/ml of methionine to prepare
protoplasts, and transformation was carried out, whereby a
transformant of pASD2, that is, ATCC 21660/pASD2 was
obtained.  It was confirmed by the same procedure as above
that the transformant harbored the plasmid.

Threonine production tests of the transformant and its
parent strain were carried out in the following manner.
First, 0.5 ml of a seed culture obtained by shaking
culture in NB medium at 30°C for 16 hours was inoculated
in 5 ml of a production medium T1 containing 100 g of
glucose, 20 g of $(NH_4)_2SO_4$, 0.5 g of $KH_2PO_4$, 0.5 g of
$K_2HPO_4$, 1 g of $MgSO_4 \cdot 7H_2O$, 10 mg of $FeSO_4 \cdot 7H_2O$, 10 mg of
$MnSO_4 \cdot 4\text{-}6H_2O$, 100 µg of biotin, 100 mg of methionine, and
20 g of calcium carbonate in 1 ℓ of water, and adjusted
to pH 7.2, and cultured with shaking at 30°C for 72 hours.
After culturing, the culture filtrate was subjected to
paper chromatography, and the amount of the L-threonine
produced was determined by colorimetry using ninhydrin
coloration after paper chromatography.  The results are
shown in Table 2, and establish that threonine-producing
ability is increased by the recombinant plasmid pASD2
containing the gene coding for ASD.

## TABLE 2

| Strain | L-threonine (mg/ml) |
| --- | --- |
| <u>Corynebacterium</u> <u>glutamicum</u> ATCC 21660 | 6.3 |
| <u>Corynebacterium</u> <u>glutamicum</u> ATCC 21660/pASD2 | 7.2 |

(5) Production of isoleucine by <u>Corynebacterium glutamicum</u> K40 containing pASD2

The isoleucine-producing strain <u>Corynebacterium glutamicum</u> K40, FERM BP-455 was cultured in SSM medium to prepare protoplasts, and transformation was carried out in the same manner as step (2) to obtain a transformant of pASD2, that is, K40/pASD2. It was confirmed in the same manner as above that the transformant harbored the plasmid.

Isoleucine production tests of the transformant and its parent strain were carried out in the same manner as step (4) above using a production medium Il consisting of the same composition as the production medium Tl, except that methionine is excluded. The results are shown in Table 3, and confirm that isoleucine-productivity is increased by pASD2 plasmid.

## TABLE 3

| Strain | L-isoleucine (mg/ml) |
| --- | --- |
| <u>Corynebacterium glutamicum</u> K40 | 1.2 |
| <u>Corynebacterium glutamicum</u> K40/pASD2 | 1.7 |

## Example 2

(1) Cloning of a gene coding for AAT of <u>Corynebacterium glutamicum</u>

Chromosome DNA was isolated in the same manner as step (1) of Example 1, except that the chromosome DNA of <u>Coryne-bacterium glutamicum</u> ATCC 31833 was used as the source of DNA. As a vector plasmid, shuttle plasmid pCE53 of

Escherichia coli and Corynebacterium glutamicum as used in Example 1 was prepared in the same manner as step (1) of Example 1.

The restriction enzyme reaction was carried out in the same manner as step (1) of Example 1 using the isolated chromosome DNA and the pCE53 plasmid DNA, and then T4 ligase reaction was carried out. The ligase reaction mixture was used for transformation. As the recipient, an aminotransferase-multideficient and host-specificity restriction-deficient mutant strain DG30 (F⁻ thr leu thi proA argE hisG recB recC sbcB hsdS hppT ilvE tyrB aspC) [J. Bacteriol. 130, 429 (1977)] derived from Escherichia coli K12 was used. The DG30 strain was deposited as Escherichia coli K59 with the FRI under the Budapest Treaty as FERM BP-900 on September 4, 1985. Competent cells of the strain were prepared according to the method of M. Dagert, et al., Gene, 6, 23 (1979).

To 0.2 ml of a suspension containing $10^9$/ml competent cells was added 100 µℓ of the above-prepared ligase reaction mixture and incubation was carried out for 10 minutes under ice-cooling. The reaction mixture was warmed at 37°C for 5 minutes and 2 ml of LB medium was added. The mixture was then incubated at 30°C for 120 minutes, and the cells were washed twice with physiological saline solution by centrifugation. The cells were spread on M9 plate medium containing 50 µg/ml each of ampicillin, threonine, leucine, isoleucine, valine, proline, arginine, histidine, phenylalanine and tyrosine. Each colony grown on the M9 plate medium was spread on LB plate medium containing 100 µg/ml ampicillin or 20 µg/ml tetracycline, and then colonies grown on the medium containing ampicillin but incapable of growing on the medium containing tetracycline were selected.

A plasmid was isolated from the selected transformants according to the above-mentioned method of G. An, et al. By digestion with various restriction enzymes and agarose gel electrophoresis, it was determined that the plasmid, pAT1, obtained from one of the transformants was a plasmid wherein a BglII DNA fragment of about 6.3 kb was inserted at the single BamHI cleavage site of pCE53 as is illustrated in Fig. 2.

A transformant of the K59 strain after retransformation with plasmid pAT1 showed that aspartic acid requirement which is based on aminotransferase-deficiency was restored to non-aspartic acid-requirement. And when AAT activity was measured according to the method of Gelfand, et al. [J. Bacteriol. 130, 429 (1977)], AAT activity was not detected in the parent strain K59, while AAT activity was detected in the retransformant. These results established that the AAT gene originating from Corynebacterium glutamicum was cloned into the pAT1 plasmid.

(2) Incorporation of pAT1 plasmid into Corynebacterium glutamicum RH6 strain

Plasmid pAT1 was introduced into Corynebacterium glutamicum RH6 strain in the same manner as step (2) of Example 1. The plasmid was isolated from the kanamycin resistant transformant obtained in the same manner as step (2) of Example 1. Analysis by digestion with various restriction enzymes and agarose gel electrophoresis established that the plasmid had the same structure as pAT1.

(3) Production of lysine by Corynebacterium glutamicum RH6 strain containing pAT1

L-lysine production tests of the transformant RH6/pAT1 obtained by the above steps and its parent strain containing no plasmid were carried out. First, 0.5 ml of a seed culture obtained by shaking culture in NB medium at 30°C for 16 hours was inoculated into 5 ml of the production medium L1, and cultured with shaking at 30°C for 72 hours. After culturing, the amount of L-lysine produced in the culture filtrate was determined by colorimetry according to the acid copper - ninhydrin method [J. Biol. Chem., 199, 91 (1952)]. The results are shown in Table 4 and establish that the recombinant plasmid pAT1 containing the gene coding for AAT has an effect of increasing L-lysine productivity.

## TABLE 4

| Strain | L-lysine (mg/ml) |
|---|---|
| Corynebacterium glutamicum RH6 | 14.8 |
| Corynebacterium glutamicum RH6/pAT1 | 16.8 |

(4) Production of threonine by Corynebacterium glutamicum ATCC 21660 containing pAT1

In the same manner as step (2) of Example 1, the threonine-producing strain Corynebacterium glutamicum ATCC 21660 was cultured in SSM medium containing 200 µg/ml methionine to prepare protoplasts, and transformation was carried out, whereby the transformant of pAT1, ATCC 21660/pAT1, was obtained. It was confirmed in the same manner as above that the transformant harbored the plasmid.

Threonine production tests of the transformant and its parent strain were carried out in the following manner. First, 0.5 ml of a seed culture obtained by shaking culture in NB medium at 30°C for 16 hours was inoculated in 5 ml of a production medium T1, and subjected to shaking culture at 30°C for 72 hours. After culturing, the culture filtrate was subjected to paper chromatography, and the amount of L-threonine produced was determined by colorimetry using ninhydrin coloration after paper chromatography. The results are shown in Table 5 and confirm that threonine-producing ability is increased by the recombinant plasmid pAT1 containing the gene coding for AAT.

TABLE 5

| Strain | L-threonine (mg/ml) |
| --- | --- |
| Corynebacterium glutamicum ATCC 21660 | 6.3 |
| Corynebacterium glutamicum ATCC 21660/pAT1 | 7.4 |

(5) Production of isoleucine by Corynebacterium glutamicum K40 containing pAT1

The isoleucine-producing strain Corynebacterium glutamicum K40 FERM BP-455 was cultured in SSM medium to prepare protoplasts and transformation was carried out in the same manner as step (2) of Example 1, whereby a transformant of pAT1, K40/pAT1, was obtained. It was confirmed in the same manner as above that the transformant harbored the plasmid.

Isoleucine production tests of the transformant and its parent strain were carried out in the same manner as step

- 22 -

(4) above using a production medium I1.  The results are shown in Table 6 and confirm that isoleucine productivity is increased by pAT1 plasmid.

<div align="center">TABLE 6</div>

| Strain | L-isoleucine (mg/ml) |
|---|---|
| Corynebacterium glutamicum K40 | 1.2 |
| Corynebacterium glutamicum K40/pAT1 | 1.8 |

## Claims

1. A process for producing an amino acid selected from the group consisting of L-lysine, L-threonine and L-isoleucine, which comprises transforming a microorganism belonging to the genus Corynebacterium or Brevibacterium with a recombinant DNA comprising an autonomously replicable vector DNA and a DNA fragment containing the gene responsible for the synthesis of aspartate semialdehyde dehydrogenase or aspartate aminotransferase of a microorganism belonging to the genus Corynebacterium or Brevibacterium in a medium until recoverable amounts of L-lysine, L-threonine or L-isoleucine are accumulated in the culture broth and recovering the amino acid therefrom.

2. A process according to Claim 1, wherein said vector is selected from the group consisting of pCG1, pCG2, pCG4, pCG11, pCE51, pCE52, pCE53, pCE54, pCB101.

3. A recombinant DNA which comprises a vector DNA and a foreign DNA fragment containing the gene responsible for the synthesis of aspartate semialdehyde dehydrogenase or aspartate aminotransferase originating from a microorganism belonging to the genus Corynebacterium or Brevibacterium.

4. A microorganism belonging to the genus Corynebacterium or Brevibacterium which harbors a replicable recombinant DNA comprising an autonomously replicable vector DNA and a DNA fragment containing the gene responsible for the synthesis of aspartate semialdehyde dehydrogenase or aspartate aminotransferase originating from a microorganism belonging to the genus Corynebacterium or Brevibacterium.

FIG. 1

# FIG.2

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:

FERM BP- 898

FERM BP- 899

FERM BP - 900